Europäisches Patentamt

European Patent Office

Office européen des brevets

(19)

(11) Veröffentlichungsnummer: **0 309 896**

**A2**

(12) **EUROPÄISCHE PATENTANMELDUNG**

(21) Anmeldenummer: 88115526.1

(22) Anmeldetag: 22.09.88

(51) Int. Cl.⁴: **C07C 67/08 , C07C 69/24 , C07C 69/75 , C07C 69/533 , C07C 69/44 , C07C 69/614 , C07C 69/78**

(30) Priorität: 30.09.87 DE 3732995

(43) Veröffentlichungstag der Anmeldung:
05.04.89 Patentblatt 89/14

(84) Benannte Vertragsstaaten:
**AT BE CH DE FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Weber, Jürgen, Dr. Dipl.-Chem.**
**Bunsenstrasse 17**
**D-4200 Oberhausen 11(DE)**
Erfinder: **Lappe, Peter, Dr. Dipl.-Chem.**
**Eickenhof 34**
**D-4220 Dinslaken(DE)**
Erfinder: **Springer, Helmut, Dipl.-Ing.**
**Drostenkampstrasse 34**
**D-4200 Oberhausen 11(DE)**

(54) **Verfahren zur Herstellung von Carbonsäuremethylestern.**

(57) Die Erfindung betrifft ein Verfahren zur Herstellung von Carbonsäuremethylestern durch Umsetzung von Mono- oder Dicarbonsäuren mit mehr als 5 Kohlenstoffatomen und Methanol in Gegenwart saurer Katalysatoren bei Temperaturen oberhalb 100° C.

EP 0 309 896 A2

## Verfahren zur Herstellung von Carbonsäuremethylestern

Die Erfindung betrifft ein Verfahren zur Herstellung von Carbonsäuremethylestern aus Monocarbonsäuren mit mehr als 5 C-Atomen im Molekül und Methanol. Hierzu werden die Reaktanten in Gegenwart eines Katalysators bei Temperaturen, die 100°C und mehr betragen, jedoch unterhalb des Siedepunktes des resultierenden Esters liegen, umgesetzt.

Der gängigste Prozeß zur Herstellung von Carbonsäureestern ist die unmittelbare Umsetzung von Carbonsäuren mit Alkoholen. Sie führt zu einem Gleichgewicht, in dem nebeneinander Alkohol, Säure, Ester und Wasser vorliegen. Die Einstellung des Gleichgewichts wird durch Katalysatoren beschleunigt.

Diese Arbeitsweise ist in der Regel nur dann wirtschaftlich, wenn das Veresterungsgleichgewicht möglichst weit zugunsten der Esterbildung verschoben wird. Geeignete Maßnahmen, dieses Ziel zu erreichen, sind die Anwendung eines großen Überschusses eines der Ausgangsstoffe oder die Entfernung eines der Produkte aus dem Reaktionsgemisch. Üblicherweise setzt man den Alkohol im Überschuß ein oder man entfernt das Reaktionswasser oder den Ester aus dem Gleichgewicht.

Die Abtrennung des Reaktionswassers kann z.B. durch Zusatz eines Trockenmittels, das das Wasser bindet oder bevorzugt durch azeotrope Destillation mit Hilfe von Schleppmitteln erfolgen.

Bei dem Verfahren der extraktiven Veresterung wird der gebildete Ester mit Hilfe eines Lösungsmittels selektiv aus dem Reaktionsgemisch herausgelöst.

Zur Herstellung von Carbonsäuremethylestern ist die Entfernung des Reaktionswassers durch azeotrope Destillation nicht geeignet. Methanol destilliert nämlich mit dem Schleppmittel in solcher Menge über, daß keine Phasentrennung im Wasserabscheider erfolgt.

Nach einem in J. Org. Chem. 24, 261 (1959) beschriebenen Verfahren gewinnt man Carbonsäuremethylester durch Umsetzung von Carbonsäuren mit Methanol in Gegenwart von Acetondimethylacetal. Das Dimethylacetal wirkt hierbei als wasserbindendes Mittel und als Methanollieferant. Setzt man Adipinsäure, Methanol und Acetondimethylacetal im molaren Verhältnis von 4 : 5 : 8 um, so erhält man Adipinsäuredimethylester in 94 %iger Ausbeute. Die Wirtschaftlichkeit dieser Arbeitsweise wird dadurch beeinträchtigt, daß die Verwendung von Acetondimethylacetal die Rohstoffkosten steigert und die Bildung von Aceton im Verlauf der Reaktion den Destillationsaufwand erhöht.

Nach dem Verfahren von Clinton und Laskowski (J.Amer.Chem.Soc. 70, 3135 (1948)), erhitzt man zur Herstellung von Methylestern eine Carbonsäure und Methanol unter Rückfluß in Gegenwart von Schwefelsäure als Katalysator und Methylen- oder Ethylenchlorid, ohne das Reaktionswasser zu entfernen. Darauf wird die organische, den Ester enthaltende Phase abgetrennt und aufgearbeitet. Diese Arbeitsweise erfordert nicht nur den Einsatz von chlorierten Kohlenwasserstoffen als Lösungsmittel für den Ester, sondern auch lange Reaktionszeiten von 6 bis 15 Stunden.

In der DE-OS 20 50 678 wird die Herstellung von Estern aus Carbonsäuren und Alkoholen in Gegenwart inerter organischer Lösungsmittel und Veresterungskatalysatoren beschrieben. Als inerte organische Lösungsmittel verwendet man Kohlenwasserstoffe mit einem Siedepunkt von -10 bis 200°C und als Katalysatoren Säuren oder Stoffe, die im wäßrigen Medium Säuren bilden, zusammen mit wäßrigen Salzlösungen. Nach den Beispielen erfordert die Herstellung von Methylestern gesättigter Carbonsäuren Reaktionszeiten von 10 Stunden (Propionsäure) bis 60 Stunden (Valeriansäure), wobei die Esterausbeuten nur 68 % bzw. 66 % betragen. Die langen Reaktionszeiten, der Einsatz von Kohlenwasserstoffen als Extraktionsmittel und die unbefriedigenden Ausbeuten stehen einer Anwendung dieser Verfahrensvariante entgegen.

Andere Verfahren zur Gewinnung von Carbonsäuremethylestern wurden eher zur Anwendung im Labor als im technischen Maßstab entwickelt. Hierzu gehören die Veresterung mit Diazomethan oder mit dem $BF_3$-Methanol-Reagens. Auch die Verwendung von Molekularsieben (Harrison et al, Chemistry and Industry (1968), S. 1568) zur Adsorption des Reaktionswassers hat in die industrielle Technik keinen Eingang gefunden.

Der Erfindung liegt die Aufgabe zugrunde, die aufgezeigten Nachteile des Standes der Technik zu vermeiden und ein allgemein anwendbares Verfahren zur Herstellung von Methylestern höherer Carbonsäuren zu entwickeln, das bei einfacher Reaktionsführung die Gewinnung der reinen Verbindungen unter wirtschaftlichen Bedingungen und in hoher Ausbeute ermöglicht.

Diese Aufgabe wird gelöst durch ein Verfahren zur Herstellung von Carbonsäuremethylestern durch Umsetzung von gesättigten oder ungesättigten, geradkettigen oder verzweigten Mono- oder Dicarbonsäuren mit mehr als 5 Kohlenstoffatomen im Molekül mit überschüssigem Methanol in Gegenwart eines sauren Katalysators. Es ist dadurch gekennzeichnet, daß man zu einem Gemisch aus Carbonsäure und Katalysator bei Temperaturen von 100 bis 150°C, jedoch unterhalb des Siedepunkts des resultierenden Esters, Methanol gibt, wobei man je Äquivalent Carbonsäure etwa 1 bis etwa 6 Mol Methanol anwendet.

Obgleich die Reaktionstemperatur deutlich oberhalb der Siedetemperatur des Methanols liegt, gelingt es nach dem erfindungsgemäßen Verfahren überraschenderweise, Carbonsäuremethylester bei einfacher Reaktionsführung und mit einem begrenzten Methanolüberschuß in sehr hohen Ausbeuten leicht herzustellen. Wegen des niedrigen Siedepunktes von Methanol war zu erwarten gewesen, daß der Alkohol in einem solchen Maße aus dem Reaktionsgemisch ausgetragen wird, daß nur Teilumsätze erzielt werden und der überwiegende Anteil der Carbonsäure unverändert zurückerhalten wird.

Als Ausgangsstoffe können nach dem erfindungsgemäßen Verfahren geradkettige oder verzweigte, gesättigte oder ungesättigte aliphatische, araliphatische oder aromatische Mono- oder Dicarbonsäuren mit mehr als 5 Kohlenstoffatomen eingesetzt werden. Beispiele für solche Säuren sind Heptansäure, Dipropylessigsäure, 2-Ethylhexansäure, Cyclohexansäure, 2-Hexensäure, Adipinsäure, Phenylessigsäure, Benzoesäure. Die Carbonsäuren können als einheitliche Verbindungen, aber auch in Form von Gemischen aus zwei oder mehr Säuren verwendet werden.

Als Veresterungskatalysatoren eignen sich vornehmlich starke ein- oder mehrbasische, nicht flüchtige anorganische oder organische Säuren, wie Schwefelsäure, Phosphorsäure, Methansulfonsäure, Ethansulfonsäure, Chlorschwefelsäure, p-Toluolsulfonsäure, aber auch saure Salze wie Natriumhydrogensulfat. Besonders geeignet ist konzentrierte Schwefelsäure.

Ein charakteristisches Merkmal des erfindungsgemäßen Verfahrens ist die Umsetzung der Reaktanten bei Temperaturen, die 100° C oder mehr betragen, aber unterhalb des Siedepunktes des gebildeten Esters liegen. Als geeignet haben sich Temperaturen zwischen 100 und 150° C erwiesen. Zweckmäßig ist es, bei 110 bis 130 und insbesondere 115 bis 125° C zu arbeiten.

Methanol wird im Überschuß eingesetzt. Je Äquivalent Carbonsäure wendet man etwa 1 bis etwa 6 Mol Methanol an. Vorzugsweise läßt man 2,0 bis 5,0 und insbesondere etwa 3,0 bis 4,0 Mol Methanol auf ein Äquivaltent Carbonsäure einwirken.

Von der als Katalysator besonders geeigneten, konzentrierten Schwefelsäure setzt man je Mol Carbonsäure 0,1 bis 3, insbesondere 0,5 bis 1,5 g ein.

Zur praktischen Durchführung der neuen Arbeitsweise legt man in einem Reaktor Carbonsäure und Katalysator vor und dosiert über eine Zulaufleitung unter Rühren des Gemisches Methanol kontinuierlich zu. Unter den Bedingungen des erfindungsgemäßen Verfahrens destilliert aus dem Reaktor kontinuierlich ein Gemisch ab, das im wesentlichen aus Wasser und Methanol und daneben geringen Mengen des gebildeten Esters besteht. Die Hauptmenge des Esters verbleibt im Reaktor. Sie wird bei absatzweiser Reaktionsführung nach beendeter Umsetzung dem Reaktor entnommen. Bei kontinuierlicher Reaktion zieht man stetig soviel Produkt enthaltendes Reaktionsgemisch ab, daß im Reaktor ein konstantes Flüssigkeitsvolumen aufrecht erhalten wird. Bei dieser Arbeitsweise muß dem Reaktor nicht nur Methanol zudosiert werden, sondern es sind auch die Anteile Carbonsäure und Katalysator zu ergänzen, die mit dem Reaktionsprodukt ausgetragen werden. Die Zuführung dieser drei Komponenten kann getrennt und bevorzugt als Gemisch erfolgen. Das Reaktionsprodukt wird in bekannter Weise durch Waschen mit Wasser vom Katalysator befreit und anschließend destillativ aufgearbeitet. Weitere Estermengen können aus dem im wesentlichen aus Wasser und Methanol bestehenden, während der Umsetzung auftretenden Destillat, gewonnen werden.

Die Ausbeuten an Carbonsäuremethylester sind sehr hoch. Sie liegen bei diskontinuierlicher Reaktionsführung zumeist über 95 % (bezogen auf eingesetzte Säure) der theoretisch zu erwartenden Menge.

Die nach dem erfindungsgemäßen Verfahren erhaltenen Produkte sind farblos und weisen Carbonsäuremethylester-Gehalte von mehr als 99 % auf.

In den nachfolgenden Beispielen wird die neue Arbeitsweise näher erläutert. Selbstverständlich ist aber nicht beabsichtigt, die Erfindung auf diese speziellen Ausführungsformen zu beschränken.


Beispiel 1: Herstellung von n-Heptansäuremethylester


In einem mit Rührer, Innenthermometer, Einleitungsrohr und einer 12 cm-Vigreuxkolonne mit Claisenbrücke ausgestatteten 1 l-Kolben werden 390,6 g n-Heptansäure (3,0 Mol) und 3,0 g konzentrierte Schwefelsäure vorgelegt und auf 125° C erhitzt. Mit Hilfe einer Pumpe dosiert man anschließend durch das Einleitungs-Tauchrohr während 6 Stunden insgesamt 384,0 g Methanol (12,0 Mol) zu. Bei einer Kopftemperatur von 66 bis 86° C fallen innerhalb dieses Zeitraumes 393,5 g Destillat an. Das Sumpfprodukt wird zur Abtrennung des Katalysators bei etwa 40° C mit Wasser gewaschen. Es verbleiben 380,5 g. Destillat und Sumpfprodukt setzen sich wie folgt zusammen:

|  | Destillat | Sumpfprodukt |
|---|---|---|
| Wasser | 13,60 | 0,02 |
| Methanol | 70,80 | 2,53 |
| Methylester | 15,35 | 97,23 |
| n-Heptansäure | 0,12 | - |
| Sonstige | 0,13 | 0,22 |

Die Ausbeute an n-Heptansäuremethylester beträgt 99,4 % der Theorie, bezogen auf eingesetzte n-Heptansäure.

Beispiel 2: Kontinuierliche Herstellung von 2-Ethylhexansäuremethylester

In einem 1 l-Kolben, der mit einem Bodenventil versehen und mit Rührer, Innenthermometer und Claisenbrucke einschließlich Vorlage ausgestattet ist, gibt man 173,0 g eines Gemisches aus 598,4 g 2-Ethylhexansäure, 397,8 g Methanol und 3,8 g konz. Schwefelsäure (Einsatzgemisch). Der Kolbeninhalt wird 30 min bei 120 °C gerührt. Dabei abdestillierende Anteile werden in einer Vorlage gesammelt. Unter Beibehaltung der Temperatur von 120 °C führt man dem Kolben kontinuierlich eine solche Menge des Einsatzgemisches zu, daß unter Berücksichtigung der abdestillierenden und der durch das Bodenventil abgezogenen Anteile, der Reaktorinhalt konstant ein Volumen von 300 bis 320 ml hat. Nach Einstellung des Gleichgewichts ergibt sich bei 6 h Versuchsdauer folgende Bilanz:

Kopftemperatur (°C)      71 - 74
Sumpftemperatur (°C)      120
Reaktorinhalt (ml)      300 - 320
Raumgeschwindigkeit      $\frac{V}{V \cdot h}$      1,26-1,34
Einsatz (g)      2075,4
Destillat (g)      859,8
Sumpfabzug (g)      1215,6

Destillat und Sumpfabzug (nach Wäsche) haben folgende Zusammensetzung:

|  | Destillat | Sumpfabzug |
|---|---|---|
| Methylester | 13,70 | 65,10 |
| 2-Ethylhexansäure | - | 31,45 |
| Methanol | 73,88 | 0,03 |
| Wasser | 12,30 | 1,50 |
| Rest | 0,12 | 1,92 |

Beispiel 3: Herstellung von Dipropylessigsäuremethylester

Die Durchführung der Umsetzung und die Katalysatorabtrennung erfolgt wie in Beispiel 1 beschrieben.

Säureeinsatz :      432,6 g Dipropylessigsäure (3,0 Mol)
Katalysator :      3,0 g konzentriertes $H_2SO_4$
Versuchsdauer :      6 Stunden
Methanolzugabe :      64,0 g/Std. (2,0 Mol)
Sumpftemperatur:      125 °C
Kopftemperatur :      69-81 °C
Destillatmenge :      401,4 g
Sumpfprodukt :      414,6 g (katalysatorfrei)

Produktzusammensetzung:

|  | Destillat | Sumpfprodukt |
|---|---|---|
| Wasser | 12,90 | 0,11 |
| Methanol | 70,70 | 1,76 |
| Methylester | 15,07 | 96,00 |
| Dipropylessigsäure | 1,20 | 2,00 |
| Sonstige | 0,12 | 0,13 |

Die Ausbeute an Dipropylessigsäuremethylester beträgt 96,6 % der Theorie, bezogen auf eingesetzte Dipropylessigsäure.

Beispiel 4: Herstellung von Cyclohexancarbonsäuremethylester

Die Durchführung der Umsetzung und die Katalysatorabtrennung erfolgt wie in Beispiel 1 beschrieben.

Säureeinsatz :       384,6 g Cyclohexancarbonsäure (3,0 Mol)
Katalysator :       3,0 g konzentriertes $H_2SO_4$
Versuchsdauer :       6 Stunden
Methanolzugabe :       64,0 g/Std. (2,0 Mol)
Sumpftemperatur:       125 °C
Kopftemperatur :       67-85 °C
Destillatmenge :       367,6 g
Sumpfprodukt :       401,0 g (katalysatorfrei)

Produktzusammensetzung:

|  | Destillat | Sumpfprodukt |
|---|---|---|
| Wasser | 13,65 | 0,12 |
| Methanol | 76,22 | 2,83 |
| Methylester | 9,40 | 95,92 |
| Cyclohexancarbonsäure | 0,22 | 0,33 |
| Sonstige | 0,51 | 0,74 |

Die Ausbeute an Cyclohexancarbonsäuremethylester beträgt 98,2 % der Theorie, bezogen auf eingesetzte Cyclohexancarbonsäure.

Beispiel 5: Herstellung von 2-Hexensäuremethylester

Die Durchführung der Umsetzung und die Katalysatorabtrennung erfolgt wie in Beispiel 1 beschrieben.

Säureeinsatz :       342,6 g 2-Hexensäure (3,0 Mol)
Katalysator :       3,0 g konzentriertes $H_2SO_4$
Versuchsdauer :       6 Stunden
Methanolzugabe :       64,0 g/std. (2,0 Mol)
Sumpftemperatur:       125 °C
Kopftemperatur :       67-85 °C
Destillatmenge :       386,0 g
Sumpfprodukt :       340,0 g (katalysatorfrei)

Produktzusammensetzung:

|  | Destillat | Sumpfprodukt |
|---|---|---|
| Wasser | 13,52 | - |
| Methanol | 72,89 | 2,90 |
| Methylester | 13,51 | 93,80 |
| 2-Hexensäure | - | 3,10 |
| Sonstige | 0,08 | 0,20 |

Die Ausbeute an 2-Hexensäuremethylester beträgt 96,6 % der Theorie, bezogen auf eingesetzte 2-Hexensäure.

Beispiel 6: Herstellung von Adipinsäuremethylester

Die Durchführung der Umsetzung und die Katalysatorabtrennung erfolgt wie in Beispiel 1 beschrieben.

Saureeinsatz :      219,0 g Adipinsäure (1,5 Mol)
Katalysator :      3,0 g konzentriertes $H_2SO_4$
Versuchsdauer :      6 Stunden
Methanolzugabe :      64,0 g/Std. (2,0 Mol)
Sumpftemperatur:      125 °C
Kopftemperatur :      67-88 °C
Destillatmenge :      335,7 g
Sumpfprodukt :      267,3 g (katalysatorfrei)

Produktzusammensetzung:

|  | Destillat | Sumpfprodukt |
|---|---|---|
| Wasser | 15,54 | 0,20 |
| Methanol | 83,19 | 4,24 |
| Methylester | 1,02 | 93,89 |
| Adipinsäure | - | 1,56 |
| Sonstige | 0,25 | 0,11 |

Die Ausbeute an Adipinsäuremethylester beträgt 97,6 % der Theorie, bezogen auf eingesetzte Adipinsäure.

Beispiel 7: Herstellung von Phenylessigsäuremethylester

Die Durchführung der Umsetzung und die Katalysatorabtrennung erfolgt wie in Beispiel 1 beschrieben.

Säureeinsatz :      408,6 g Phenylessigsäure (3,0 Mol)
Katalysator :      3,0 g konzentriertes $H_2SO_4$
Versuchsdauer :      6 Stunden
Methanolzugabe :      64,0 g/Std. (2,0 Mol)
Sumpftemperatur:      125 °C
Kopftemperatur :      67-89 °C
Destillatmenge :      346,0 g
Sumpfprodukt :      446,6 g (katalysatorfrei)

Produktzusammensetzung:

|  | Destillat | Sumpfprodukt |
|---|---|---|
| Wasser | 15,20 | 0,25 |
| Methanol | 78,64 | 3,68 |
| Methylester | 5,67 | 95,90 |
| Phenylessigsäure | 0,20 | 0,10 |
| Sonstige | 0,29 | 0,17 |

Die Ausbeute an Phenylessigsäuremethylester beträgt 99,4 % der Theorie, bezogen auf eingesetzte Phenylessigsäure.

Beispiel 8: Herstellung von Benzoesäuremethylester

Die Durchführung der Umsetzung und die Katalysatorabtrennung erfolgt wie in Beispiel 1 beschrieben.

Säureeinsatz : 366,4 g Benzoesäure (3,0 Mol)
Katalysator : 3,0 g konzentriertes $H_2SO_4$
Versuchsdauer : 6 Stunden
Methanolzugabe : 64,0 g/Std. (2,0 Mol)
Sumpftemperatur: 125 °C
Kopftemperatur : 69-81 °C
Destillatmenge : 355,4 g
Sumpfprodukt : 395,0 g (katalysatorfrei)

Produktzusammensetzung:

|  | Destillat | Sumpfprodukt |
|---|---|---|
| Wasser | 14,67 | 0,21 |
| Methanol | 78,74 | 2,51 |
| Methylester | 6,43 | 95,61 |
| Benzoesäure | 0,02 | 1,59 |
| Sonstige | 0,14 | 0,08 |

Die Ausbeute an Benzoesäuremethylester beträgt 98,0 % der Theorie, bezogen auf eingesetzte Benzoesäure.

Beispiel 9: Herstellung von 2-Ethylhexansäuremethylester

Die Durchführung der Umsetzung und die Katalysatorabtrennung erfolgt wie in Beispiel 1 beschrieben.

Säureeinsatz : 432,6 g 2-Ethylhexansäure (3,0 Mol)
Katalysator : 3,0 g konzentriertes $H_2SO_4$
Versuchsdauer : 6 Stunden
Methanolzugabe : 64,0 g/Std. (2,0 Mol)
Sumpftemperatur: 125 °C
Kopftemperatur : 67-84 °C
Destillatmenge : 415,5 g
Sumpfprodukt : 400,3 g (katalysatorfrei)

Produktzusammensetzung:

|  | Destillat | Sumpfprodukt |
|---|---|---|
| Wasser | 12,42 | 0,18 |
| Methanol | 67,50 | 2,59 |
| Methylester | 18,50 | 95,65 |
| 2-Ethylhexansäure | 1,22 | 1,28 |
| Sonstige | 0,37 | 0,30 |

Die Ausbeute an 2-Ethylhexansäuremethylester beträgt 96,9 % der Theorie, bezogen auf eingesetzte 2-Ethylhexansäure.

**Ansprüche**

1.) Verfahren zur Herstellung von Carbonsäuremethylestern durch Umsetzung von gesättigten oder ungesättigten, geradkettigen oder verzweigten Mono- oder Dicarbonsäuren mit mehr als 5 Kohlenstoffatomen im Molekül mit überschüssigem Methanol in Gegenwart eines sauren Katalysators, dadurch gekennzeichnet, daß man zu einem Gemisch aus Carbonsäure und Katalysator bei Temperaturen von 100 bis 150° C, jedoch unterhalb des Siedepunktes des resultierenden Esters Methanol gibt, wobei man je Äquivalent Carbonsäure etwa 1 bis etwa 6 Mol Methanol anwendet.

2.) Verfahren nach Anspruch 1, dadurch gekennzeichnet, daß die Zugabe des Methanols bei Temperaturen von 110 bis 130° C, insbesondere von 115 bis 125° C erfolgt.

3.) Verfahren nach Anspruch 1 oder 2, dadurch gekennzeichnet, daß man je Äquivalent Carbonsäure 2,0 bis 5,0, insbesondere etwa 3,0 bis 4,0 Mol Methanol anwendet.

4.) Verfahren nach einem oder mehreren der Ansprüche 1 bis 3, dadurch gekennzeichnet, daß als saurer Katalysator konzentrierte Schwefelsäure verwendet wird.

5.) Verfahren nach Anspruch 4, dadurch gekennzeichnet, daß je Mol Carbonsäure 0,1 bis 3 g, insbesondere 0,5 bis 1,5 g konzentrierte Schwefelsäure eingesetzt werden.